# EUROPÄISCHE PATENTANMELDUNG

(11) **EP 1 834 601 A1**
(43) Veröffentlichungstag der Anmeldung: **19.09.2007**
(21) Anmeldenummer: 07100834.6
(22) Anmeldetag: 19.01.2007
(51) Int. Cl.: A61B 19/00

(54) **Stoßwellenbehandlungsvorrichtung mit einer Stauvorrichtung mit Schiebedeckel**

(30) Priorität: 16.03.2006 DE 102006012228
(71) Anmelder: SIEMENS AKTIENGESELLSCHAFT, 80333 München (DE)
(72) Erfinder: Fehre, Jens, 91353, Hausen (DE); Lanski, Markus, 90782, Fürth (DE); Mahler, Matthias, 91058, Erlangen (DE)

(57) **Zusammenfassung**

Die Erfindung betrifft eine Stoßwellenbehandlungsvorrichtung (10) mit einem Stoßwellenkopf (12), der während einer Stoßwellenbehandlung Stoßwellen emittiert, wobei der Stoßwellenkopf (12) an einer beweglichen Halteeinrichtung (13) angeordnet ist und sich über die Halteeinrichtung (13) auf einer eine Oberseite aufweisende Stativeinheit (11) abstützt, und mit einer schließbaren Staueinrichtung (20). Indem die Staueinrichtung (20) eine die Oberseite der Stativeinheit (11) wenigstens teilweise ausbildende Deckeleinheit (21) aufweist, und dass wenigstens ein Führungsmittel (22) an der Stativeinheit (11) zur Verschiebung der Deckeleinheit (21) mit einer vertikalen und/oder einer horizontalen Bewegungskomponente relativ zur Stativeinheit (11) vorgesehen ist, wird eine Stativeinheit einer Stoßwellenhandlungsvorrichtung bereitgestellt, welche ein sicheres Ablegen und Verstauen von Objekten gewährleistet und dabei platzsparend ausgebildet ist.

## Beschreibung

Die Erfindung betrifft eine Stoßwellenbehandlungsvorrichtung mit einem Stoßwellenkopf, der während einer Stoßwellenbehandlung Stoßwellen emittiert, wobei der Stoßwellenkopf an einer beweglichen Halteeinrichtung angeordnet ist und sich über die Halteeinrichtung auf einer eine Oberseite aufweisende Stativeinheit abstützt, und mit einer schließbaren Staueinrichtung.

Die Behandlung bzw. Therapie eines Untersuchungsobjekts mittels Stoßwellen findet in mehreren Gebieten der modernen Medizin Anwendung. Beispielsweise werden Stoßwellenbehandlungen in der Orthopädie oder auch in der Lithotripsie, d.h. Zertrümmerung von Konkrementen, wie etwa Gallen-, Harnleiter- und Nierensteine, eingesetzt. Ein anderes Einsatzgebiet für Stoßwellenbehandlungen ist die Schmerztherapie. Bei den genutzten Stoßwellen handelt es sich um mediumgebundene Longitudinalwellen, also Schallwellen, welche in der Regel einen Ultraschall-Frequenzbereich aufweisen. Heute kommt häufig die extrakorporale Stoßwellentherapie (ESWT) bzw. extrakorporale Stoßwellenlithotripsie (ESWL) zum Einsatz. Dabei werden die Stoßwellen mittels eines in der Regel elektroakustischen Wandlers im Stoßwellenkopf erzeugt, anschließend in ein ebenfalls im Stoßwellenkopf befindliches Wasserreservoir eingekoppelt und von dort mittels eines Koppelmittels in das Untersuchungsobjekt eingeleitet. Im Wasserreservoir des Stoßwellenkopfes werden zuvor die Stoßwellen mittels einer Linse - häufig aus Kunststoff - auf einen Fokus gebündelt, dessen Position mit der Position des zu behandelnden Punkts bzw. Zone im Untersuchungsobjekt übereinstimmt.

Je nach durchzuführender Behandlung am Untersuchungsobjekt werden unterschiedliche Energien pro Zeiteinheit in das Untersuchungsobjekt mittels der Stoßwellen eingebracht bzw. appliziert. Bei der Lithotripsie werden Stoßwellen mit hoher Energie in das Untersuchungsobjekt eingekoppelt, um die im Fokus liegenden Strukturen, z.B. Harnleitersteine oder Nierensteine, zu zertrümmern. Bei der Schmerztherapie werden im Wesentlichen niedrigere Energien genutzt. Ziel hierbei ist es mittels der Stoßwellen eine Druck- und Zugbelastung des schmerzenden Gewebes zu erreichen, um den Stoffwechsel an diesen Stellen zu stimulieren und Schmerzen zu lindern. Eine orthopädische Anwendung, bei welcher die verwendeten Energien der Stoßwellen zwischen denen der lithotriptischen Anwendung und der schmerztherapeutischen Anwendung liegen, ist bspw. die Kalkschulter. Dabei wird versucht Kalkablagerungen im Schultergelenk zu entfernen, um die Beweglichkeit der Schulter zu erhalten.

Zur Durchführung einer Stoßwellenbehandlung wird eine stationäre oder verfahrbare Stoßwellenbehandlungsvorrichtung genutzt. Diese weist eine Stativeinheit, ein Halteeinrichtung und einen Stoßwellenkopf auf, welcher sich mittels der Halteeinrichtung auf der Stativeinheit abstützt. Die Halteeinrichtung umfasst in der Regel eine Mehrzahl an Halteelementen, welche gegeneinander gedreht und gegebenenfalls verschoben werden können. Dadurch kann die Position des Stoßwellenkopfs in einem weiten Raumbereich geändert werden und damit der Stoßwellenkopf bspw. als Untertischstoßwellenkopf oder Obertischstoßwellenkopf flexibel auf die Anforderungen der Behandlung eingestellt werden. Der Fokus der Stoßwellenbehandlungsvorrichtung wird in der Regel in den Behandlungspunkt des Untersuchungsobjekts gelegt. Der Behandlungspunkt wird in der Regel mittels bildgebender Ultraschalltechnik oder Röntgentechnik ermittelt und das Untersuchungsobjekt und/oder die Stoßwellenbehandlungsvorrichtung anhand der Untersuchungen entsprechend positioniert und ausgerichtet.

Aus der Offenlegungsschrift DE 41 35 177 A1 ist eine Therapieeinrichtung zur Behandlung eines Lebewesens mit fokussierten akustischen Wellen bekannt, welches eine Quelle fokussierter, akustischer Wellen aufweist sowie ein Koppelmittel zur Einleitung der akustischen Wellen in ein zu behandelndes Lebewesen. Nachteil der offenbarten Therapieeinrichtung ist es, dass keinerlei Ablage- und/oder Staumöglichkeiten an der Therapieeinrichtung vorgesehen sind, welche zum sicheren Ablegen oder Verstauen von vom medizinischen Personal oder von einem Patienten benötigten Mitteln bzw. Objekten nutzbar sind.

In der Offenlegungsschrift DE 10 2004 010 005 A1 ist eine Anlage zur bildgestützten Stoßwellenbehandlung offenbart. Die Anlage weist einen Stoßwellenkopf auf und eine Trageeinrichtung für den Stoßwellenkopf, welcher am Freiende eines mit einem Fixierende an der Trageeinrichtung verbunden Auslegers angeordnet ist. Auch bei dieser stationären Anlage ist keine ausgewiesene Ablagemöglichkeit bzw. ausgewiesener Stauraum vorgesehen, um vom medizinischen Personal benötigten Mittel bzw. Objekte zu verstauen.

Zudem ist aus der Broschüre: Dornier Compact Sigma von Dornier MedTech, im Internet unter der folgenden URL-Adresse: "http://www.dornier.com/americas/english_gb/products/lithotri pters/compactSigma/brochure.htm" auffindbar, ein modularer Lithotripter offenbart, welcher eine Staueinrichtung in Form einer konventionellen Schublade aufweist. Nachteil der offenbarten Staueinrichtung ist es, dass die gesamte Staueinrichtung bei deren Öffnung bewegt wird, so dass der Inhalt der Staueinrichtung eine Beschleunigung erfährt und damit die Gefahr einer Beschädigung des Inhalts der Staueinrichtung gegeben ist. Weiter ist eine Öffnung der Staueinrichtung nur manuell durchführbar, so dass stets eine länger andauernde, kontrollierte Bewegung der öffnenden Person erforderlich ist, um die Staueinrichtung zu öffnen.

Der Erfindung liegt die Aufgabe zugrunde, eine Stoßwellenbehandlungsvorrichtung der eingangs genannten Art sowie eine Stativeinheit einer medizinischen Behandlungsvorrichtung, bereitzustellen, welche ein sicheres Ablegen und Verstauen von Objekten gewährleistet und dabei platzsparend ausgebildet ist.

Die Aufgabe wird bei einer gattungsgemäße Stoßwellenbehandlungsvorrichtung der eingangs genannten Art dadurch gelöst, dass die Staueinrichtung eine die Oberseite der Stativeinheit wenigstens teilweise ausbildende Deckeleinheit aufweist, und dass wenigstens ein Führungsmittel an der Stativeinheit zur Verschiebung der Deckeleinheit mit einer vertikalen und/oder einer horizontalen Bewegungskomponente relativ zur Stativeinheit vorgesehen ist.

Durch die erfindungemäße Staueinrichtung kann zusätzlich zu herkömmlich angeordneten Staueinrichtungen, wie etwa aus dem Stand der Technik bekannt, die Staukapazität in oder an der Stoßwellenbehandlungsvorrichtung vergrößert werden. Insbesondere geschieht dies dadurch, dass die den Stauraum mindestens teilweise umgebende Deckeleinheit mehrere Funktionalitäten aufweist. Die Führungsmittel können im Rahmen der Erfindung auf vielfältige Art und Weise realisiert werden, bspw. als Schienenführung.

In einer vorteilhaften Ausgestaltung der Erfindung ist die Deckeleinheit einteilig ausgebildet. Dadurch kann ein starrer Körper realisiert werden, der sich durch Zugkraft oder Druckkraft besonders einfach verschieben lässt. Insbesondere können bei der Herstellung der Deckeleinheit bspw. Gießverfahren, Spritzverfahren oder Tiefziehverfahren angewendet werden, wodurch die Arbeitsschritte zur Herstellung der Deckeleinheit verringert werden können. Des Weiteren kann die Führung der Verschiebung durch einfache Führungsmittel ermöglicht werden. Die Deckeleinheit kann transparent oder nicht transparent ausgebildet sein. Eine transparente Deckeleinheit erlaubt einen Blick in den von der Staueinrichtung umfassten Stauraum, wodurch eine schnelle Evaluierung des Inhalts ohne Öffnung der Staueinrichtung erfolgen kann. Eine nicht transparent ausgebildete Deckeleinheit weist gerade den Vorteil auf, dass der Stauraum der Staueinrichtung durch Dritte nicht einsehbar ist. In der Staueinrichtung können dann bspw. auch Dokumente mit vertraulichem Inhalt - etwa Patientenakten - gelagert werden.

In einer vorteilhaften Ausgestaltung der Erfindung ist die Deckeleinheit als eine im Wesentlichen horizontale Ablageeinrichtung ausgebildet. Dadurch erhält die Deckeleinheit der Staueinrichtung neben der Abgrenzung eines Stauraums der Staueinrichtung von einer Umgebung eine weitere Funktion, nämlich als gut zugängliche, einfache und sichere Ablagemöglichkeit für Objekte, bspw. Ortungszubehör für die Lokalisierung der Behandlungszone. Die durch die Führungsmittel geführte Verschiebung der Deckeleinheit erfolgt dabei derart, dass die Ablageeinrichtung während der Verschiebung zwar die Position, d.h. ihren Ort, aber nicht ihre räumliche Lage, d.h. ihre Ausrichtung im Raum, ändert. Daher ist die Ablageeinrichtung während der gesamten Verschiebung im Wesentlichen horizontal angeordnet, so dass auf der Ablageeinrichtung abgelegte Objekte nicht ohne weiteres verrutschen. Beim Öffnen der Staueinrichtung durch Verschieben der Deckeleinheit werden Objekte, welche sich auf der Ablageeinrichtung bei Öffnung der Staueinrichtung befinden, zwar mitverschoben, sind jedoch jederzeit sicher gelagert. Die Ablageeinrichtung kann dabei auf die Erfordernisse der an einem Untersuchungsobjekt durchzuführenden Behandlung angepasst werden. Die Ablageeinrichtung kann z.B. besondere Einrichtungen zur sicheren und gegebenenfalls sterilen Lagerung medizinischer Instrumente oder Therapiemittel vorsehen. Vorzugsweise weist die Ablageeinrichtung eine rutschsichere, ausgedehnte Ablagefläche auf.

In einer weiteren vorteilhaften Ausführungsform der Erfindung weist die Deckeleinheit einen erhöhten Seitenrand auf. Ein erhöhter Seitenrand der Deckeleinheit umfasst dabei mehrere Vorteile. Grundsätzlich kann dabei nur ein Seitenrand erhöht ausgebildet sein oder auch eine Mehrzahl an Seitenrändern der Deckeleinheit. Die Erhöhung einer Mehrzahl an Seitenrändern ist insbesondere von Vorteil bei der Ausbildung der Deckeleinheit als Ablageeinrichtung. Sollten abgelegte Objekte durch eine horizontale Relativbeschleunigung des Objekts zur Ablageeinrichtung auf der Ablageeinrichtung verrutschen, so wird durch einen erhöhten Seitenrand vermieden, dass das Objekt die Ablageeinrichtung verlässt. Die erhöhten Seitenränder dienen somit als Rutschstopp. Ein erhöhter Seitenrand weist zudem den Vorteil auf, dass dieser als Griff zur manuellen Verschiebung der Deckeleinheit gegen die Stativeinheit genutzt werden kann. Dadurch entfällt das Anbringen eines zusätzlichen Griffs zur Verschiebung der Deckeleinheit, was Kosten einspart und wodurch man eine größtmögliche, sichere Ablagefläche erhält. Des Weiteren kann der Griff schlüssig in das Gesamtkonzept der Staueinrichtung bzw. der Deckeleinheit eingeformt werden, indem er bspw. während der Herstellung der Deckeleinheit, z.B. durch Anwendung eines Gießverfahrens, miterzeugt wird.

In einer vorteilhaften Ausführungsvariante der Erfindung ist wenigstens bei der Verschiebung der Deckeleinheit aus einem geschlossenen Zustand in einen geöffneten Zustand der Staueinrichtung ein zu überwindender Verschiebewiderstand vorgesehen. Der Verschiebewiderstand kann im Hinblick auf unterschiedliche physikalische Größen justiert werden. So z.B. hinsichtlich der Position der Deckeleinheit relativ zur Stativeinheit, der Geschwindigkeit mit welcher die Deckeleinheit relativ zur Stativeinheit verschoben wird oder aber die Kraft, welche zum Verschieben der Deckeleinheit aufgewendet wird. Dadurch wird vermieden, dass die Staueinrichtung versehentlich geöffnet wird, zu schnell geschlossen wird oder ähnliches. Dies erhöht die Sicherheit für die im Stauraum der Staueinrichtung als auch für die auf der Ablageeinrichtung der Staueinrichtung gelagerten Objekte.

In einer weiteren vorteilhaften Ausgestaltung der Erfindung ist die Deckeleinheit motorisch verschiebbar. Dadurch kann das medizinische Personal trotz anderer Aktivität - z.B. Halten medizinischer Instrumente - die Deckeleinheit einfach öffnen. Dazu kann eine Bedieneinrichtung vorgesehen werden, welche bei Betätigung bzw. Bedienung durch das medizinische Personal die Ansteuerung einer Antriebseinrichtung bewirkt, welcher die Deckeleinheit relativ zur Stativeinheit verschiebt. Durch erneute Betätigung der Bedieneinrichtung kann die Deckeleinheit wieder geschlossen werden. So ist eine kontrollierte Öffnung und Schließung der Staueinrichtung möglich, ohne dass das medizinische Personal die Verschiebung steuern muss. Dadurch kann die Aufmerksamkeit des medizinischen Personals in größerem Maße als bei einer manuellen Bedienung der Deckeleinheit, beim Untersuchungsobjekt verbleiben.

In einer weiteren vorteilhaften Ausführungsvariante der Erfindung ist die Deckeleinheit in einer Zwischenstellung zwischen dem geschlossenen Zustand und einem vollständig geöffneten Zustand der Staueinrichtung feststellbar. Dadurch kann ein größtmögliches Maß an Flexibilität für den Zugang zum Stauraum und zur Ablageeinrichtung erreicht werden. Die Feststellbarkeit kann durch Feststellmittel erfolgen oder auch nur durch die Reibungskraft zwischen Deckeleinheit und Führungsmittel. Insbesondere können die Feststellmittel als Rastverbindung ausgebildet sein, deren Haltekraft gegebenenfalls einstellbar ist.

In einer weiteren vorteilhaften Ausführungsform der Erfindung ist die Staueinrichtung von der Stativeinheit entfernbar. Dadurch kann ein modulares Konzept für die Stoßwellenbehandlungsvorrichtung ermöglicht werden. So kann nach Entfernen der Staueinrichtung bspw. eine Ein-/Ausgabeeinrichtung oder andere für die Behandlung erforderliche Einrichtungen auf die vormals von der Staueinrichtung eingenommen Position platziert werden. Dies erhöht die Einsatzflexibilität der gesamten Stoßwellenbehandlungsvorrichtung.

In einer weiteren vorteilhaften Ausgestaltung der Erfindung ist die Staueinrichtung abschließbar. Indem die Staueinrichtung abschließbar ist, d.h. die Deckeleinrichtung durch Bedienung einer Verschließeinrichtung im Wesentlichen nicht mehr relativ zur Stativeinheit verschiebbar ist, können bspw. medizinische Mittel direkt an der Stoßwellenbehandlungsvorrichtung mitgeführt werden. Mögliche medizinische Mittel sind bspw. Kontrastmittel für die Lokalisierung des Behandlungspunkts im Untersuchungsobjekt. Durch Halterungen, welche innerhalb der Staueinrichtung angeordnet sein können, kann eine weitere Erhöhung der Sicherheit für bspw. medizinische Mittel erreicht werden. So kann etwa eine Flaschenhalterung für bspw. Kontrastmittel beinhaltende Flaschen bzw. Ampullen vorgesehen werden.

In einer weiteren vorteilhaften Ausführungsform der Erfindung ist die Stativeinheit auf Rollelementen gelagert. Dadurch kann die gesamte Stoßwellenbehandlungsvorrichtung verfahren werden. Dies ist häufig erforderlich, da eine Stoßwellenbehandlungsvorrichtung öfters in Kombination mit einer weiteren medizinischen Vorrichtung, z.B. einer Röntgenvorrichtung, verwendet wird. Dadurch kann es erforderlich sein, die Position der weiteren medizinischen Vorrichtung und der Stoßwellenbehandlungsvorrichtung relativ zueinander zu ändern, um bspw. eine verbesserte Zugänglichkeit zum Untersuchungsobjekt zu erreichen. Dies kann einfach erreicht werden, indem die Stoßwellenbehandlungsvorrichtung auf Rollelementen gelagert wird. Rollelemente können auf unterschiedlichste Art und Weise ausgebildet sein - etwa als zylindrische Rollen, Kugeln usw.

In einer weiteren vorteilhaften Ausführungsvariante der Erfindung ist wenigstens ein Teil eines Stativgriffs der Stativeinheit derart ausgebildet, dass die Deckeleinheit mittels des Teils des Stativgriffs führbar ist. Dadurch können die zusätzlich zum Stativgriff angebrachten Führungsmittel in ihren Abmessungen verkleinert werden, was Kosten einspart, und die Führung der Deckeleinheit wenigstens teilweise vom Stativgriff oder wenigstens eines seiner Teile übernommen werden. Der Stativgriff dient somit nicht nur als Angriffsbereich zur Verschiebung der gesamten Stativeinheit, sondern auch als Führungsmittel für die Deckeleinheit und entfaltet damit eine kombinatorische Wirkung. Dazu ist es in der Regel erforderlich, wenigstens einen Teil des Stativgriffs derart am Stativ anzuordnen, dass dieser Teil einen ähnlichen oder gleichen Winkel mit der Oberflächennormalen der Deckeleinheit einschließt wie die unabhängig davon vorgesehenen Führungsmittel. In einem speziellen Fall können die gesamten Führungsmittel zur Führung der Deckeleinheit als Teil des Stativgriffs ausgebildet sein.

Die zugrunde liegende Aufgabe wird zudem gelöst von einer Stativeinheit einer medizinischen Behandlungsvorrichtung, mit einer Oberseite, mit einer schließbaren Staueinrichtung, wobei an der Stativeinheit eine Halteeinrichtung gelagert ist, an welcher eine medizinische Behandlungseinrichtung angeordnet ist, wobei die Staueinrichtung eine die Oberseite der Stativeinheit wenigstens teilweise ausbildende Deckeleinheit aufweist, und dass wenigstens ein Führungsmittel an der Stativeinheit zur Verschiebung der Deckeleinheit mit einer vertikalen und/oder einer horizontalen Bewegungskomponente relativ zur Stativeinheit vorgesehen ist. Die Staueinrichtung kann somit auch für Stativeinheiten, welche nicht Bestandteil einer Stoßwellenbehandlungsvorrichtung sind, vorgesehen werden. Dies betrifft insbesondere verfahrbare und nicht verfahrbare Röntgenvorrichtungen und weitere medizinischen Behandlungsvorrichtungen, welche aufgrund ihrer kompakten und platzsparenden Konzeption einen Mangel an Stauraum und gegebenenfalls Ablageflächen aufweisen.

Weitere Vorteile der Erfindung ergeben sich aus einem Ausführungsbeispiel, welches nachfolgend anhand der Zeichnungen erläutert wird, in deren
- FIG 1: eine Seitenansicht einer erfindungsgemäßen Stoßwellenbehandlungsvorrichtung in geschlossenem Zustand,
- FIG 2: eine Seitenansicht einer erfindungsgemäßen Stoßwellenbehandlungsvorrichtung in geöffnetem Zustand,
- FIG 3: eine Draufsicht einer erfindungsgemäßen Stoßwellenbehandlungsvorrichtung in geschlossenem Zustand,
- FIG 4: eine Draufsicht einer erfindungsgemäßen Stoßwellenbehandlungsvorrichtung in geöffnetem Zustand

schematisch dargestellt sind.

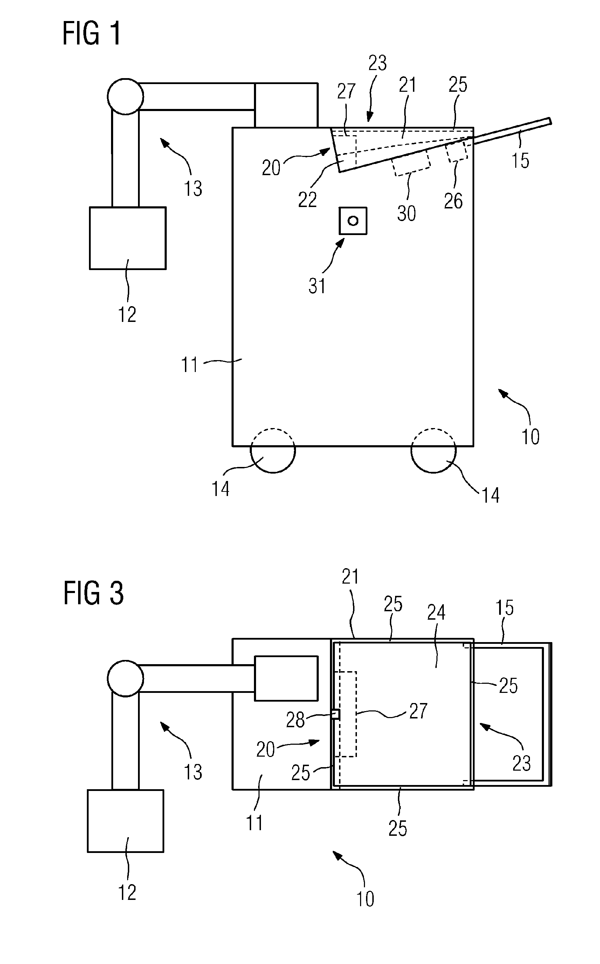

FIG 1 bis FIG 4 zeigen eine Stoßwellenbehandlungsvorrichtung 10, welche eine Stativeinheit 11, einen Stoßwellenkopf 12 und eine den Stoßwellenkopf 12 mit der Stativeinheit 11 verbindende Halteeinrichtung 13 aufweist. Die Halteeinrichtung 13 umfasst eine Mehrzahl an Haltelementen, welche gegeneinander und relativ zur Stativeinheit 11 beweglich gelagert sind. Dadurch kann die Position des Stoßwellenkopfs 12 stets auf die Erfordernisse einer Stoßwellenbehandlung angepasst werden.

Des Weiteren ist die Stativeinheit 11 auf Rollelementen 14 gelagert, so dass diese verfahrbar ausgebildet ist. Die Stativeinheit 11 der Stoßwellenvorrichtung 10 ist in der Regel möglichst klein und platzsparend ausgebildet, da die verfahrbare Stoßwellenbehandlungsvorrichtung 10 häufig in Kombination mit einer nicht dargestellten röntgentechnischen Vorrichtung - etwa einer C-Bogen-Röntgenvorrichtung - kombiniert wird, um einen Behandlungspunkt eines Untersuchungsobjekts zu lokalisieren. Durch den Einsatz mehrerer medizintechnischer Vorrichtungen an einem Untersuchungsobjekt kann es zur gegenseitigen Einschränkung der Bewegungsmöglichkeiten durch die räumliche Ausdehnung der jeweils anderen Vorrichtung bzw. Vorrichtungen kommen. Derartige Einschränkungen werden versucht zu verringern, indem unter anderem die den medizinischen Vorrichtungen zugehörigen Stativeinheit bzw. Stativeinheiten möglichst kompakt und platzsparend ausgebildet sind.

Die Stativeinheit 11 der Stoßwellenbehandlungsvorrichtung 10 weist eine Staueinrichtung 20 auf, welche mehrere Funktionalitäten umfasst und somit gut an das Konzept einer kompakten Stativeinheit 11 angepasst werden kann. Die Staueinrichtung 20 umfasst eine Deckeleinheit 21, welche wenigstens teilweise die Oberseite der Stativeinheit 11 ausbildet. Die Deckeleinheit 21 ist an der Stativeinheit 11 auf zwei in die Seitenwände der Stativeinheit 11 eingearbeitete Führungsschienen 22 gelagert. Damit ist eine formschlüssige Verbindung der Deckeleinheit 21 mit den Führungsschienen 22 gegeben. Durch eine formschlüssige Verbindung können zusätzliche Führungsmittelkomponenten eingespart werden, was Kostenvorteile mit sich bringt. Jedoch kann als Führungsmittel bspw. auch wenigstens eine metallische Führungsschiene zwischen der Stativeinheit 11 und der Deckeleinheit 21 vorgesehen werden. Dies stellt gegebenenfalls eine kostenintensivere Ausführung der Führungsmittel dar, weist jedoch andere technische Vorteile auf, z.B. Erzeugung eines einstellbaren Verschiebewiderstandes für die Verschiebung der Deckeleinheit 21 relativ zur Stativeinheit 11.

Die Deckeleinheit 21 wird mittels Führungsschienen 22 geführt und kann relativ zur Stativeinheit 11 verschoben werden. Dadurch wird der im geschlossenen Zustand von der Deckeleinheit 21 umgebene Stauraum der Staueinrichtung 20 freigegeben. Die Verschiebung der Deckeleinheit 21 relativ zur Stativeinheit 21 kann schrittweise oder kontinuierlich erfolgen. Bspw. können für eine schrittweise Verschiebung der Deckeleinheit die Führungsschiene 22 ein Wellenprofil aufweisen. Die Staueinrichtung 20 kann zudem mehrere geöffnete Zustände, welche sich in der durch die Verschiebung bewirkte Position der Deckeleinheit 21 unterscheiden, einnehmen. Im Ausführungsbeispiel schließen die Führungsschienen 22 mit der senkrecht auf der Oberfläche der Stativeinheit 11 bzw. Deckeleinheit 21 stehenden Oberflächennormalen einen Winkel von etwa 60 Grad ein. Der eingeschlossene Winkel ist jedoch nicht auf 60 Grad beschränkt. Grundsätzlich sind eingeschlossene Winkel zwischen Führungsschiene 22 und Oberflächennormale von 0 Grad bis 90 Grad möglich.

Die Oberseite der Deckeleinheit 21 der Staueinrichtung 20 ist im Ausführungsbeispiel als Ablageeinrichtung 23 ausgebildet, wodurch die Deckeleinheit 21 eine weitere Funktionalität erhält. Zum einen umgibt die Deckeleinheit 21 im geschlossenen Zustand der Staueinrichtung 20 den Stauraum und grenzt diesen damit von der Umgebung ab. Zum anderen kann die Deckeleinheit 21 als Ablageeinrichtung 23 genutzt werden. Die Ablageeinrichtung 23 weist vorteilhafterweise eine oder mehrere rutschfeste, sterilisierbare Ablageflächen 24 auf. Die Ablageeinrichtung 23 wird durch erhöhte Seitenränder 25 in horizontaler Richtung begrenzt, um ein Rutschen eines abgelegten Objekts von der Ablageeinrichtung 23 zu vermeiden, z.B. bei einem plötzlichen Abbremsen der auf Rollelemente 14 bewegten Stativeinheit 11. Zudem kann eine innere Unterteilung der Ablageeinrichtung 23 in mehrere Ablageflächen 24 durch nicht dargestellte Abgrenzungseinrichtungen vorgesehen werden.

Vorzugsweise sind diejenigen Seitenränder 25, welche senkrecht zur betragsmäßig größeren Verschiebungsrichtungskomponente stehen, als Griffe ausgebildet. Die Griffe können auf vielfältige Art und Weise ausgeführt werden. Die betragsmäßig größere Verschiebungsrichtungskomponente ist vom eingeschlossenen Winkel der Führungsschienen 22 und der Oberflächennormalen der Deckeleinheit 21 bzw. der Stativeinheit 11 abhängig. Bei einem eingeschlossenen Winkel von 45 Grad ist die horizontale Verschiebungskomponente betragsmäßig gleich der vertikalen Verschiebungskomponente. Durch die Ausbildung wenigstens eines Seitenrands der Deckeleinheit als Griff wird in besonders einfacher und kostengünstiger Weise wenigstens ein Angriffsbereich für das Personal zur Verschiebung der Deckeleinheit 21 bereitgestellt.

Ein weiterer Vorteil, welcher sich aus der in FIG 1 bis FIG 4 gezeigten Anordnung der Führungsschienen 22 relativ zur Oberflächennormalen der Oberseite der Stativeinheit 11 ergibt, liegt darin, dass die Ablageeinrichtung 23 höhenverstellbar ist, so dass die vertikale Positionskomponente der Ablageeinrichtung 23 in einem gewissen Bereich der Größe der benutzenden Person der Stoßwellenbehandlungsvorrichtung 10 angepasst werden kann. Dieser Vorteil tritt für alle von der Oberflächennormale der Deckeleinheit 21 bzw. der Stativeinheit 11 mit den Führungsschienen 22 eingeschlossene Winkel ungleich 90 Grad auf.

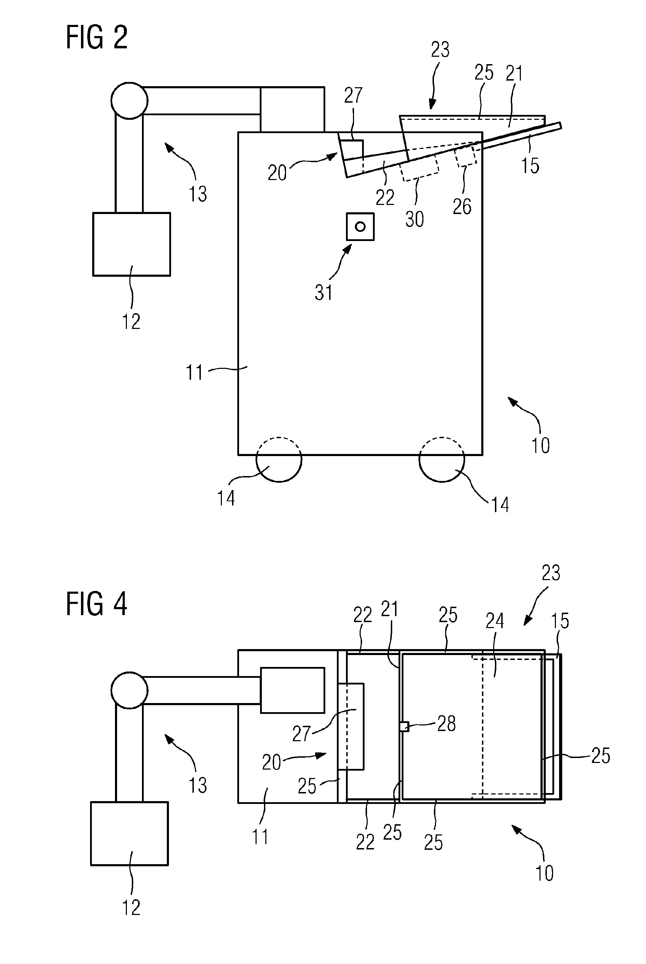

FIG 1 und FIG 2 zeigt außerdem ein an der Stativeinheit 11 angeordnetes Widerstandsmittel 26, welches im Ausführungsbeispiel als Federelement ausgebildet ist und einen Verschiebewiderstand bei der Verschiebung der Deckeleinheit 21 der Staueinrichtung 20 vom geschlossenen Zustand in einen geöffneten Zustand bewirkt. Jedoch sind auch andere Ausbildungen von Widerstandsmitteln 26 denkbar sowie Anordnungen an den Führungsschienen 22 oder auch der Staueinrichtung 20. Die Erzeugung des Verschiebwiderstands kann auch über eine elektrische und/oder elektromagnetische Einrichtung ermöglicht werden. Das Widerstandsmittel 26 verhindert ein versehentliches Öffnen und/oder Schließen der Staueinrichtung 20, indem ein zu überwindender Verschiebewiderstand mittels des Widerstandsmittels 26 erzeugt wird. Vorteilhafterweise sind neben dem Widerstandsmittel Feststellmittel vorgesehen. Die Feststellmittel können auch mit dem Widerstandsmittel 26 identisch sein oder kombiniert werden. Die Feststellmittel dienen dazu, eine bestimmte Position der Deckeleinheit 21 - insbesondere bei einem geöffneten Zustand der Staueinrichtung 20 - relativ zur Stativeinheit 11 beizubehalten. Die Deckeleinheit 21 wird bspw. manuell aus dem geschlossenen Zustand in einen (teil-) geöffneten Zustand geführt. Soll die Deckeleinheit 21 über einen längeren Zeitraum in dieser Position verbleiben, so können Feststellmittel zur Feststellung bzw. Arretierung der Deckeleinheit in dieser Position vorgesehen werden. Feststellmittel sind vorteilhafterweise insbesondere dann vorzusehen, wenn die vom von Oberflächennormale der Oberseite der Stativeinheit 11 und den Führungsschienen 22 eingeschlossenen Winkel abhängige Reibungskraft der Deckeleinheit 21 und Führungsschiene 22 eine Arretierung der Deckeleinheit in einer bestimmten Position nicht erlaubt.

Die Staueinrichtung 20 weist zudem eine Abschließeinrichtung 28 in Form eines Schlosses auf. Damit können auch hinsichtlich des Datenschutzes sensible Dokumente oder auch Medikamente in der Staueinrichtung 20 gelagert werden und vor einem unbefugten Zugriff geschützt werden. Das Schloss 28 ist vorzugsweise mittels einer Nummernkombination elektrisch oder mechanisch zu- und aufschließbar. Gegebenenfalls können auch andere Mittel 28 zum Verschließen der Staueinrichtung 20 vorgesehen werden - bspw. ein Fingerabdruckscanner. Dadurch entfallen mechanische Schlüssel, die sonst möglicherweise in hoher Anzahl ausgegeben werden müssten. Die Staueinrichtung 20 weist vorteilhafter eine Unterteilung 27 des Stauraums, z.B. für Dokumente - wie etwa auch die Bedienungsanleitung der Stoßwellenbehandlungsvorrichtung 10 - sowie benötigte Therapiemittel und/oder Medikamente, auf.

Die Verschiebung der Deckeleinheit 21 relativ zur Stativeinheit 11 kann gegebenenfalls auch motorisch bewirkt werden. Dazu kann eine Antriebseinheit 30 vorgesehen werden, welche die Deckeleinheit 21 entlang der Führungsschienen 22 verschiebt. Die Verschiebung kann vorteilhafterweise über eine Bedieneinrichtung 31 mindestens ausgelöst und vorteilhafterweise auch eingestellt werden. Die Bedieneinrichtung 31 kann bspw. als in die Stativeinheit 11 versenkter Knopf ausgebildet sein, wobei die Bewegung bzw. Verschiebung der Deckeleinheit 21 über die Dauer des Knopfdrucks durch das Personal erfolgt. Die Versenkung des Knopfes verhindert ein versehentliches Auslösen der Verschiebung der Deckeleinheit 21. Durch die Nutzung einer Antriebseinrichtung 30 in Verbindung mit einer Bedieneinrichtung 31 wird der manuelle Kontakt mit der Ablageeinrichtung 23 verringert und dadurch der Eintrag von Keimen auf die Ablageeinrichtung 23, auf der bspw. medizinische Instrumente gelagert werden können. Gegebenfalls kann eine Steuereinrichtung vorgesehen werden, welche derart eingerichtet ist, dass die Verschiebung der Deckeleinheit 21 steuerbar ist. Dies ist insbesondere dann zweckmäßig, wenn bereits eine Steuereinrichtung bspw. zur Steuerung des Stoßwellenkopfes 12 vorgesehen ist, und die Steuerung der Deckeleinheit 21 nur ein weiteres hinterlegtes Steuerverfahren in der Steuereinrichtung darstellt und somit keine zusätzlichen Kosten entstehen.

## Patentansprüche

1. Stoßwellenbehandlungsvorrichtung (10) mit einem Stoßwellenkopf (12), der während einer Stoßwellenbehandlung Stoßwellen emittiert, wobei der Stoßwellenkopf (12) an einer beweglichen Halteeinrichtung (13) angeordnet ist und sich über die Halteeinrichtung (13) auf einer eine Oberseite aufweisende Stativeinheit (11) abstützt, und mit einer schließbaren Staueinrichtung (20),
**dadurch gekennzeichnet, dass** die Staueinrichtung (20) eine die Oberseite der Stativeinheit (11) wenigstens teilweise ausbildende Deckeleinheit (21) aufweist, und dass wenigstens ein Führungsmittel (22) an der Stativeinheit (11) zur Verschiebung der Deckeleinheit (21) mit einer vertikalen und/oder einer horizontalen Bewegungskomponente relativ zur Stativeinheit (11) vorgesehen ist.

2. Stoßwellenbehandlungsvorrichtung nach Anspruch 1,
**dadurch gekennzeichnet, dass** die Deckeleinheit (21) einteilig ausgebildet ist.

3. Stoßwellenbehandlungsvorrichtung nach Anspruch 1 oder 2,
**dadurch gekennzeichnet, dass** die Deckeleinheit (21) als eine im Wesentlichen horizontale Ablageeinrichtung (23) ausgebildet ist.

4. Stoßwellenbehandlungsvorrichtung nach einem der Ansprüche 1 bis 3,
**dadurch gekennzeichnet, dass** die Deckeleinheit (21) einen erhöhten Seitenrand (25) aufweist.

5. Stoßwellenbehandlungsvorrichtung nach einem der Ansprüche 1 bis 4,
**dadurch gekennzeichnet, dass** wenigstens bei der Verschiebung der Deckeleinheit (21) aus einem geschlossenen Zustand in einen geöffneten Zustand der Staueinrichtung (20) ein zu überwindender Verschiebewiderstand vorgesehen ist.

6. Stoßwellenbehandlungsvorrichtung nach einem der Ansprüche 1 bis 5,
**dadurch gekennzeichnet, dass** die Deckeleinheit (21) motorisch verschiebbar ist.

7. Stoßwellenbehandlungsvorrichtung nach einem der Ansprüche 1 bis 6,
**dadurch gekennzeichnet, dass** die Deckeleinheit (21) in einer Zwischenstellung zwischen dem geschlossenen Zustand und einem vollständig geöffneten Zustand der Staueinrichtung (20) feststellbar ist.

8. Stoßwellenbehandlungsvorrichtung nach einem der Ansprüche 1 bis 7,
**dadurch gekennzeichnet, dass** die Staueinrichtung (20) von der Stativeinheit (11) entfernbar ist.

9. Stoßwellenbehandlungsvorrichtung nach einem der Ansprüche 1 bis 8,
**dadurch gekennzeichnet, dass** die Staueinrichtung (20) abschließbar ist.

10. Stoßwellenbehandlungsvorrichtung nach einem der Ansprüche 1 bis 9,
**dadurch gekennzeichnet, dass** die Stativeinheit (11) auf Rollelementen (14) gelagert ist.

11. Stoßwellenbehandlungsvorrichtung nach einem der Ansprüche 1 bis 10,
**dadurch gekennzeichnet, dass** wenigstens ein Teil eines Stativgriffs (15) der Stativeinheit (11) derart ausgebildet ist, dass die Deckeleinheit (21) mittels des Teils des Stativgriffs (11) führbar ist.

12. Stativeinheit (11) einer medizinischen Behandlungsvorrichtung (10), mit einer Oberseite und einer schließbaren Staueinrichtung (20), wobei an der Stativeinheit (11) eine Halteeinrichtung (13) gelagert ist, an welcher eine medizinische Behandlungseinrichtung (12) angeordnet ist,
**dadurch gekennzeichnet, dass** die Staueinrichtung (20) eine die Oberseite der Stativeinheit (11) wenigstens teilweise ausbildende Deckeleinheit (21) aufweist, und dass wenigstens ein Führungsmittel (22) an der Stativeinheit (11) zur Verschiebung der Deckeleinheit (21) mit einer vertikalen und/oder einer horizontalen Bewegungskomponente relativ zur Stativeinheit (11) vorgesehen ist.
